# EUROPEAN PATENT SPECIFICATION

(11) **EP 3 996 640 B1**
(45) Date of publication and mention of the grant of the patent: **12.10.2022**
(21) Application number: 20750041.4
(22) Date of filing: 08.07.2020
(51) Int. Cl.: A61F 5/453, A61F 5/455

(54) **FLUID COLLECTION DEVICES, SYSTEMS AND METHODS**
VORRICHTUNGEN, SYSTEME UND VERFAHREN ZUM SAMMELN VON FLÜSSIGKEITEN
LES DISPOSITIFS, SYSTÈMES ET MÉTHODES DE COLLECTE DE FLUIDES

(30) Priority: 11.07.2019 US 201962873045 P
(43) Date of publication of application: 18.05.2022
(73) Proprietor: Purewick Corporation, El Cajon, California 92020 (US)
(72) Inventor: JOHANNES, Ashley Marie, Atlanta, Georgia 30340 (US); CHALLA, Pranav, Atlanta, Georgia 30308 (US)
(74) Representative: Hoffmann Eitle
(86) International application number: PCT/US2020/041242
(87) International publication number: WO 2021/007345

(56) References cited:
- WO-A1-2017/210524
- WO-A1-2018/144463
- US-A1- 2018 228 642

## Description

### BACKGROUND

An individual may have limited or impaired mobility such that typical urination processes are challenging or impossible. For example, the individual may have surgery or a disability that impairs mobility. In another example, the individual may have restricted travel conditions such as those experience by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bed pans and urinary catheters have several problems associated therewith. For example, bed pans may be prone to discomfort, spills, and other hygiene issues. Urinary catheters be may be uncomfortable, painful, and may cause urinary tract infections.

US 2018/0228642 A1 discloses a urine collecting device to be disposed against the body of a female user, with a fenestration in an external covering of the device in operative relation with the urethra of the user. A tube for transporting urine away from the device has its end in fluid communication with an endcap of the device, into which urine flows via the fenestration.

Thus, users and manufacturers of fluid collection devices continue to seek new and improved devices, systems, and methods to collect urine.

### SUMMARY

The invention is defined in claim 1 below. The dependent claims are directed to optional features and preferred embodiments. Disclosed herein are fluid collection devices and methods of using fluid collection devices. A fluid collection device is disclosed which includes at least one wicking material including an outer surface. The fluid collection device includes a fluid impermeable coating adhered to at least a portion of the outer surface of the at least one wicking material, the fluid impermeable coating defining a chamber within the fluid collection device partially enclosing the at least one wicking material and a defining an opening through which a portion of the at least one wicking material is exposed, the opening configured to be positioned adjacent to a female urethra.

A fluid collection system is disclosed. The fluid collection system includes a fluid storage container configured to hold a fluid. The fluid collection system includes a fluid collection device including at least one wicking material including an outer surface. The fluid collection device includes a fluid impermeable coating adhered to at least a portion of an outer surface of the at least one wicking material, the fluid impermeable coating defining a chamber within the fluid collection device and an opening through which a portion of the at least one wicking material is exposed, the opening configured to be positioned adjacent to a female urethra or have a male urethra positioned therethrough. The fluid collection system includes a conduit disposed within the chamber, the conduit including an inlet positioned within the chamber and an outlet configured to be fluidly coupled to a vacuum source. The fluid collection system includes a vacuum source fluidly coupled to one or more of the fluid storage container or the outlet of the fluid collection device, the vacuum source configured to draw fluid from the fluid collection device.

A method to collect fluid is disclosed. The method includes positioning an opening of a fluid collection device adjacent to a female urethra or around a male urethra of a user, the opening defined by a fluid impermeable coating of the fluid collection device. The method includes receiving fluid from the female urethra into a chamber of the fluid collection device, the chamber of the fluid collection device at least partially defined by the fluid impermeable coating.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present invention, wherein identical reference numerals refer to identical or similar elements or features in different views or embodiments shown in the drawings.
**FIG. 1A** is an isometric view of a fluid collection device, according to an embodiment.
**FIG. 1B** is a cross-sectional view of the fluid collection device of **FIG. 1A** taken along the plane A-A, according to an embodiment.
**FIGS. 1C** and **1D** are cross-sectional views of the area **B** of **FIG. 1B****,** according to various embodiments.
**FIG. 2A** is an isometric view of a fluid collection device, according to an embodiment.
**FIGS. 2B** and **2C** are cross-sectional views of the fluid collection device of **FIG. 2A** taken along the plane C-C, according to various embodiments.
**FIG. 3A** is cancelled.
**FIG. 3B** is cancelled.
**FIG. 4** is a block diagram of a system for fluid collection.
**FIG. 5** is a flow diagram of a method to collect fluid.
**FIG. 6** is a flow diagram of a method to making a fluid collection device.

### DETAILED DESCRIPTION

Disclosed herein are fluid collection devices and methods of using the same. The devices and systems disclosed herein are configured to collect fluids from an individual. The fluids collected by the fluid collection devices may include at least one of urine, vaginal discharge, penile discharge, reproductive fluids, blood, sweat, or other bodily fluids.

The fluid collection devices include wicking material having a fluid impermeable coating that at least partially defines a chamber therein. The fluid impermeable coating also defines an opening extending therethrough that is configured to be positioned adjacent to a female urethra or have a male urethra positioned therethrough. The fluid collection device also includes a conduit having a channel extending between an inlet and outlet thereof. The inlet is fluidly coupled to the chamber and the outlet is configured to be fluidly coupled to a fluid storage (vessel or container) or vacuum. The fluid impermeable coatings and fluid collection devices utilizing the coatings disclosed herein provide a soft, comfortable, and relatively smooth feel for the wearer, even on sensitive areas of the anatomy and under moist conditions.

Some conventional fluid collection devices may have a fluid impermeable shell defining an outer surface of the device. The fluid impermeable shell often includes a material that has a thickness that can create discontinuities on the surface of the device or has a relatively higher material hardness or texture than the absorbing material enclosed therein. In some instances, a conventional fluid impermeable shell may have shape, thickness, and/or material composition differences (*e.g*., transitions from hard to soft materials) that can create cusps, ridges, or other geometric features at the outer surface of the fluid collection device. These features may create discomfort on the skin of a wearer during use, especially prolonged use *(e.g.,* more than an hour). For example, the transition from fluid impermeable shell material to the absorbing material therein may provide a lip or other discontinuity that creates discomfort or even sores on the skin of the wearer over time. Similarly, relatively stiffer materials in the shell may not yield to pressure exerted thereon by the skin of a wearer. Such stiffer materials may also result in discomfort or sores during prolonged use.

The fluid collection devices disclosed herein have fluid impermeable coating with one or more of a thickness, compliance, material make-up, and lack of surface features sufficient to provide a smooth comfortable feel to the wearer of the fluid collection device, even after long periods of use. For example, the fluid impermeable coating is thin enough so as not to provide a rigid or hard surface against the skin of a wearer. The fluid impermeable coating is thin enough to comply to pressure asserted thereon and provide a surface that provides little to no cusps or discontinuities. For example, the relatively thin fluid impermeable coatings disclosed herein do not create a discontinuity when transitioning from the fluid impermeable coating to the wicking material in the opening. Accordingly, the fluid collection devices herein limit or eliminate sores and discomfort compared to conventional fluid collection devices.

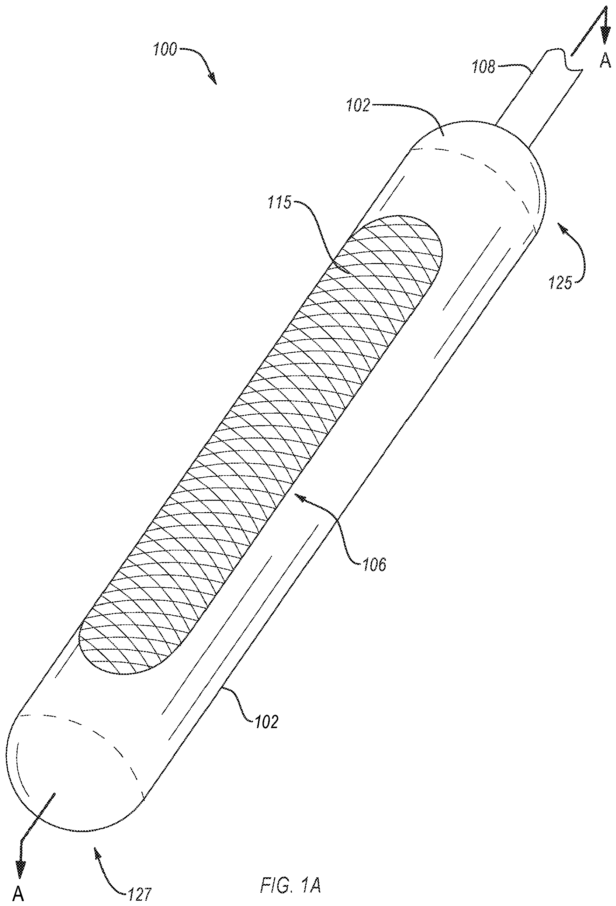

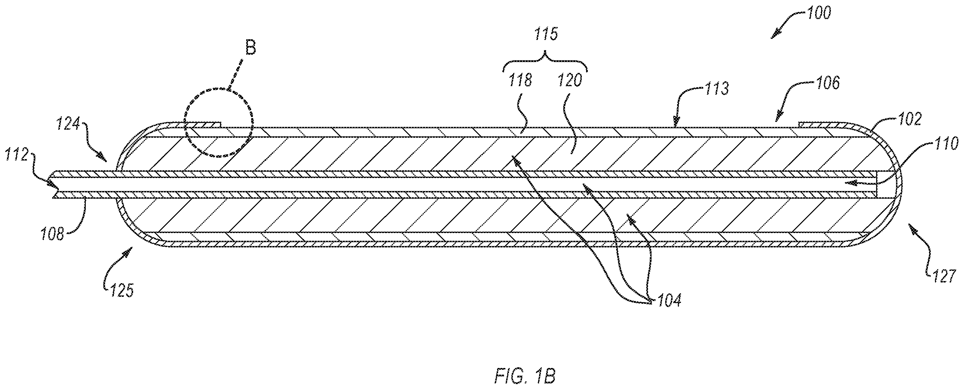

**FIG. 1A** is an isometric view of a fluid collection device 100, according to an embodiment. **FIG. 1B** is a cross-sectional view of the fluid collection device of **FIG. 1A** taken along the plane A-A, according to an embodiment. References to both **FIGS. 1A** and **1B** are made below. The fluid collection device 100 may include a fluid impermeable coating 102 and wicking material 115. The wicking material 115 may be disposed within the fluid impermeable coating 102. The fluid collection device 100 may also include a conduit 108. The conduit 108 may be at least partially disposed within wicking material 115.

The fluid impermeable coating 102 at least partially defines at least a portion of an outer surface of the fluid collection device 100. The fluid impermeable coating 102 at least partially defines a chamber 104 in the fluid collection device 100 *(e.g.,* interior region of the fluid collection device 100) and an opening 106. The opening 106 is defined by, or formed in and extends through, the fluid impermeable coating 102 thereby enables fluids to enter the chamber 104 from outside of the fluid collection device 100. The chamber 104 contains at least a portion of the wicking material 115 and conduit 108. The wicking material 115 is exposed through the opening 106. The opening 106 may be located on a portion of the fluid collection device 100 expected to be positioned adjacent to a female urethra, such as on or between the labia majora of a female user. For example, the opening 106 may allow urine to pass to the wicking material 115 from a wearer and at least temporarily retain the fluid within the chamber 104.

The fluid impermeable coating 102 at least temporarily retains or stores fluids in the chamber 104. The fluid impermeable coating 102 may be formed of a material and/or have a thickness selected to provide a relatively soft feel of the fluid collection device 100 against the skin of a wearer. The fluid impermeable coating 102 may be a thin layer of material disposed on or in an outer surface 113 of the wicking material 115.

In some embodiments, the fluid impermeable coating 102 may be formed of a material with a relatively soft feel, such as with a hardness lower than an average silicone. The fluid impermeable coatings 102 disclosed herein prevent or limit discontinuities between an outer impermeable barrier or shell and the wicking material, such as when a conventional silicone outer shell is used. The relatively thin and/or soft material of the fluid impermeable coating 102 prevents discomfort from relatively stiff or hard materials traditionally used in fluid collection devices when positioned against a wearer, provides a substantially uniform feel over the entire device, and provides a relatively soft feel at an outer surface of the device compared to devices with molded fluid impermeable barriers.

The fluid impermeable coating 102 may be formed of any suitable fluid impermeable materials, such as a fluid impermeable polymer, wax, nanocellulose, nanoparticles, another suitable material, or combinations thereof. As such, the fluid impermeable coating 102 may prevent at least some of the fluid(s) from exiting the portions of the chamber 104 that are spaced from the opening 106. The fluid impermeable coating 102 may be a hydrophobic material. For example, the fluid impermeable coating may include one or more of silicone, polypropylene, polyethylene, polyethylene terephthalate, polystyrene, polyurethane, a polycarbonate, polychloroprene, vinyl, polyvinyl chloride, latex, silanes *(e.g.,* an halogenated alkyl silane), perfluorinated polymers, etc. The fluid impermeable coating 102 may include a superhydrophobic material, such as fluorocarbon polymers, fluorosilane polymers, metal oxide polystyrene nanocomposites (*e.g.*, MnO₂ or ZnO), textured silica, carbon nanotubes, etc. The fluid impermeable coating 102 may be an elastomeric material, such as silicone-rubber. Other suitable materials include thermoplastic elastomers such as one or more of thermoplastic styrene-containing block copolymers, thermoplastic polyurethanes, thermoplastic copolyesters, thermoplastic olefins, thermoplastic polyamides, or the like.

In some examples, the fluid impermeable coating 102 may be oleophobic. In some examples, the material of the fluid impermeable coating 102 may also be composed to reduce or prevent dissolution of the fluid impermeable coating 102 in the presence of acid or basic body fluids.

In some embodiments, the fluid impermeable coating 102 may be air permeable and fluid impermeable. In such an example, the fluid impermeable coating 102 may be formed of a hydrophobic material that defines a plurality of pores sized and shaped to allow gases to pass therethrough but retain liquids.

The thickness of the fluid impermeable coating 102 may be kept to a minimum, to provide a fluid impermeable barrier, to have a smooth discontinuity-free feel, and allow the fluid collection device to have the overall feel of the outermost portion of the wicking material 115. For example, the thickness of the fluid impermeable coating 102 may be sufficiently thin to provide a tactile feel of the outermost portion of the wicking material 115 *(e.g.,* fluid permeable membrane 118). The thickness of the fluid impermeable coating 102 may be less than about 2 mm, about 10 µm to about 2 mm, about 500 µm to about 1.5 mm, about 100 µm to about 1 mm, about 1 mm to about 2 mm, about 10 µm to about 200 µm, about 10 µm to about 500 µm, about 200 µm to about 500 µm, more than about 500 µm, less than about 1 mm, less than about 500 µm, or less than about 100 µm.

In some examples, the fluid impermeable coating 102 may include multiple layers of any of the foregoing materials. For example, the fluid impermeable coating 102 may include a thin layer *(e.g.,* less than 1 mm) of latex coated in textured silica, such as on the outer side of the latex layer or the inner, chamber-facing side of the latex layer.

In an example, at least one or more portions of an outer surface of the fluid impermeable coating 102 may be formed from a soft and/or smooth material thereby reducing discomfort, chafing, or sores on the skin of the user. For example, any of the materials disclosed herein for the fluid impermeable coating 102 may be provided with one or more portions having a relative smooth texture, such as having surface roughness measured in Ra value of less than about 30 micrometers (µm), such as 0.1 µm to 30 µm, 0.5 µm to about 20 µm, 1 µm to 10 µm, 0.1 µm to 5 µm, less than 20 µm, less than 15 µm, less than 10 µm, less than 5 µm, or less than 2 µm.

In some examples, the fluid impermeable coating 102 may be relatively soft such as having a relatively low hardness *(e.g.,* a Shore A hardness value of less than 40, such as less than about 30, less than about 20, or less than about 10; and in some cases a Shore OO hardness value of less than about 50 or less than about 40). By using a relatively soft material, such as any of those described herein, the fluid impermeable coating 102 may be more comfortable than fluid collection devices that use relatively harder materials such as an outer shell made from rubber or silicone having an average hardness *(e.g.,* a Shore A hardness of 40 or more).

The fluid impermeable coating 102 may include markings thereon, such as one or more markings to aid a user in aligning the device 100 on the wearer. For example, a line on the fluid impermeable coating 102 *(e.g.,* opposite the opening 106) may allow a healthcare professional to align the opening 106 over the urethra of the wearer. The markings may include one or more of alignment guide or an orientation indicator, such as a stripe or hashes. Such markings may be positioned to align the device 100 to one or more anatomical features such as a pubic bone, etc.

As shown in **FIG. 1B****,** the fluid impermeable coating 102 may be disposed on an outermost portion of the wicking material 115. For example, the fluid impermeable coating 102 may be a layer of material adhered to an outer surface 113 of the wicking material 115.

The wicking material 115 may be disposed within the fluid impermeable coating 102. The wicking material 115 may include permeable material designed to wick or pass fluid therethrough. The permeable properties referred to herein may be wicking, capillary action, diffusion, or other similar properties or processes, and are referred to herein as "permeable" and/or "wicking." Such "wicking" may not include absorption into the wicking material. The wicking material 115 may collect the fluid that travels through the opening 106. The wicking material 115 may include more than one material, such as a plurality of materials. The plurality of materials may include a plurality of layers concentrically disposed within one another. The concentrically disposed layers of wicking materials may exhibit a gradient of wicking, such as where the innermost wicking material includes the greatest or least wicking ability of the plurality of materials. In some examples, the plurality of layers of wicking materials may be non-concentrically arranged, such as having an innermost layer of wicking material located off-center of one or more outer layers of wicking material(s) and/or fluid impermeable coating. In such examples, the conduit 108 may be non-concentrically located in the fluid collection device 100, such as in the non-concentrically located innermost layer of wicking material.

In some examples, the wicking material 115 may include one or more of a fluid permeable support 120 or a fluid permeable membrane 118. For example, the fluid collection device 100 may include a fluid permeable membrane 118 disposed in the chamber 104. The fluid permeable membrane 118 may cover at least a portion *(e.g.,* all) of the opening 106. The fluid permeable membrane 118 may be configured to wick any fluid away from the opening 106 thereby preventing the fluid from escaping the chamber 104. The fluid permeable membrane 118 may also wick the fluid generally towards an interior of the chamber 104, as discussed in more detail below. The fluid permeable membrane 118 may include any material that may wick the fluid. For example, the fluid permeable membrane 118 may include fabric, such as a gauze *(e.g.,* a silk, linen, polyester, Rayon, cotton, or blends of gauzes), another soft fabric, or another smooth fabric. The fluid permeable membrane 118 may include spun plastic fibers. In some examples, the fluid permeable membrane 118 may include an open cell foam, such as a polyester foam. Forming the fluid permeable membrane 118 from gauze, soft fabric, and/or smooth fabric may reduce chafing caused by the fluid collection device 100 rubbing against the skin of a user.

The fluid permeable support 120 is disposed in the chamber 104, such as concentrically within the fluid permeable membrane 118. The fluid permeable support 120 may support the fluid permeable membrane 118 due to the relatively malleable form of the fluid permeable membrane material 118. For example, the fluid permeable membrane 118 may be formed from a foldable, flimsy, or otherwise easily deformable material, such as a gauze type fabric composed to wick but not absorb fluids. Accordingly, the fluid permeable membrane may 118 be held in shape and place or otherwise be supported by the fluid permeable support 120. In some examples, the fluid permeable support 120 may be positioned such that the fluid permeable membrane 118 is disposed between the fluid permeable support and the fluid impermeable coating 102. As such, the fluid permeable support 120 may support and maintain the position of the fluid permeable membrane 118. The fluid permeable support 120 may include a relatively more rigid or dense material than the fluid permeable membrane 118. The fluid permeable support 120 may be formed from any fluid permeable material that is less deformable than the fluid permeable membrane 118, such as any of the materials disclosed herein for the fluid impermeable membrane 118, in a more dense or rigid form. For example, the fluid permeable support 120 may include a porous polymer (*e.g.*, nylon, polyester, polyurethane, polyethylene, polypropylene, etc.) structure or an open cell foam. The fluid permeable support 120 may include spun plastic fibers. In some examples, the fluid permeable support 120 may be formed from a natural material, such as cotton, wool, silk, or combinations thereof. In some examples, the fluid permeable support 120 may be formed from fabric, felt, gauze, or combinations thereof. The fluid permeable support 120 may be resiliently flexible to help impart resiliency and flexibility to the fluid collection device 100. In some examples, the fluid permeable support 120 may be omitted from the fluid collection device 100. For example, the wicking material 115 may include only the fluid permeable membrane 118. In some examples, the fluid permeable membrane 118 may be optional. For example, the wicking material 115 may include only the fluid permeable support 120. The fluid permeable support 120 may have a greater density than the fluid permeable membrane 118 or be may be made from a more rigid material than the fluid permeable membrane 118.

The wicking material 115 *(e.g.,* at least the fluid permeable support) may provide the structural support to maintain a general shape of the fluid collection device 100, which may be resiliently flexible. For example, the wicking material 115 may be in a substantially cylindrical shape, a substantially flat shape, a substantially prismatic shape, etc. Likewise, the materials applied thereover *(e.g.,* the fluid impermeable coating 102) may exhibit the same shape.

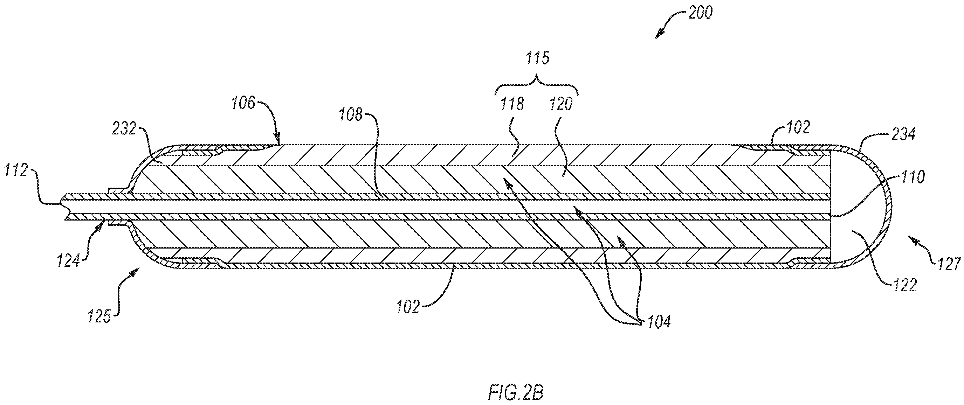

The fluid impermeable coating 102 and the wicking material 115 may be configured to receive the conduit 108 at least partially within the chamber 104. For example, at least one of the fluid permeable membrane 118 and the fluid permeable support 120 may be configured to form a space that accommodates the conduit 108. In another example, the fluid impermeable coating 102 may define an aperture sized to receive the conduit 108 *(e.g.,* at least one tube). The at least one conduit 108 may be disposed in the chamber 104 via the aperture 124, such as via an aperture formed in the first end region 132 of the fluid collection device. The aperture 124 may be sized and shaped to form an at least substantially fluid tight seal against the conduit 108 thereby substantially preventing the fluids from escaping the chamber 104. In some examples (not shown), the aperture 124 may be disposed on the second end region 134. In such examples, the conduit 108 may be disposed in only the second end region 134 with the inlet 110 being disposed in the second end region 134 *(e.g.,* the reservoir 122, **FIG. 2B**).

Locating the inlet 110 in a position of the fluid collection device that is intended to be positioned at or near a gravimetrically low point of the chamber 104 when the fluid collection device is in use enables the conduit 108 to receive more of the fluids than if inlet 110 was located elsewhere and reduces the likelihood of pooling (*e.g.,* pooling of the fluids may cause microbe growth and foul odors). For instance, the fluids in the wicking material 115 may flow in any direction due to capillary forces. However, the fluids may exhibit a preference to flow in the direction of gravity, especially when at least a portion of the wicking material 115 is saturated with the fluids.

As discussed in more detail below, the conduit 108 is sized and shaped to be coupled to, and at least partially extend between, one or more of a fluid storage container (not shown) and a vacuum source (not shown). In an example, the conduit 108 is configured to be directly connected to the vacuum source. In such an example, the conduit 108 may extend from the fluid impermeable coating 102 by at least one foot, at least two feet, at least three feet, or at least six feet. In another example, the conduit 108 is configured to be indirectly connected to at least one of the fluid storage container and the vacuum source. In some examples, the conduit 108 may be secured to a wearer's skin with a catheter securement device, such as a STATLOCK^{®} catheter securement device available from C. R. Bard, Inc., including but not limited to those disclosed in U.S. Patent Nos. 6,117,163; 6,123,398; and 8,211,063.

The term "coating" is not intended to be limited to only material disposed on an outer surface of another material, rather a coating may be an entire layer, a portion of layer, or a separate layer disposed on or in the wicking material as disclosed in more detail herein.

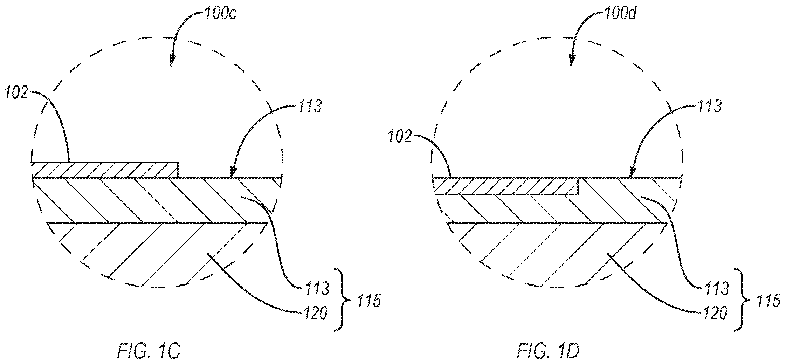

As shown in **FIGS. 1C** and **1D****,** the fluid impermeable coating 102 may be disposed on or in the wicking material 115. **FIGS. 1C** and **1D** are cross-sectional views of the area **B** of **FIG. 1B****.** As shown in **FIG. 1C****,** the fluid collection device 100c includes the fluid impermeable coating 102 disposed on the outer surface 113 of the wicking material 115 *(e.g.,* the fluid permeable membrane 118). The fluid collection device 100c is similar or identical to the fluid collection device 100 in one or more aspects. The fluid impermeable coating 102 is deposited on the surface 113 of the wicking material 115. The fluid impermeable coating 102 may be cast, coated, wrapped, sprayed, or otherwise deposited on the surface 113 to a selected thickness, such as any of the thicknesses disclosed herein. In some examples, the fluid impermeable coating may be adhered to the outermost portion of the wicking material *(e.g.,* fluid impermeable membrane), such as with an adhesive or may be interfacially self-bonded to the wicking material such as via dip coating.

In some embodiments, the fluid impermeable coating 102 may be least partially embedded or otherwise disposed in the outermost portion of the wicking material 115. As shown in **FIG. 1D****,** the fluid collection device 100d includes the fluid impermeable coating 102 embedded in the outer surface 113 of the wicking material 115 *(e.g.,* the fluid permeable membrane 118). The fluid collection device 100d is similar or identical to the fluid collection device 100 in one or more aspects. The fluid impermeable coating 102 is deposited in the outer most portion of the wicking material 115 and at least partially defines the surface 113 of the wicking material 115. In such examples, the outer surface of the fluid impermeable coating 102 may be coextensive with the outer surface 113 of the wicking material 115 as shown. In some examples (not shown), the outer surface of the fluid impermeable coating 102 may extend from within the wicking material 115 to beyond with the outer surface 113 of the wicking material 115. The fluid impermeable coating 102 may be cast, coated, sprayed, permeated, or otherwise incorporated into the wicking material 115 at the surface 113 to a selected depth therein (*e.g*., coating thickness), such as to any of the thicknesses disclosed herein. Upon application, the wicking material 115 may at least partially wick the fluid impermeable membrane material into the outer surface thereof. In such examples, the fluid impermeable coating material may be infiltrated into the wicking material 115 sufficient to cause the infiltrated portion of the wicking material to be fluid impermeable.

As shown in **FIG. 1B**, the wicking material 115 *(e.g.,* one or more of the fluid permeable membrane 118 or the fluid permeable support 120) may at least substantially completely fill portions of the chamber 104 that are not occupied by the conduit 108. For example, the wicking material 115 may fill the portions of the chamber 104 that are not occupied by the conduit 108 when the fluid impermeable coating 102 is disposed over the entire outer surface of the wicking material 115 (except for the opening 106).

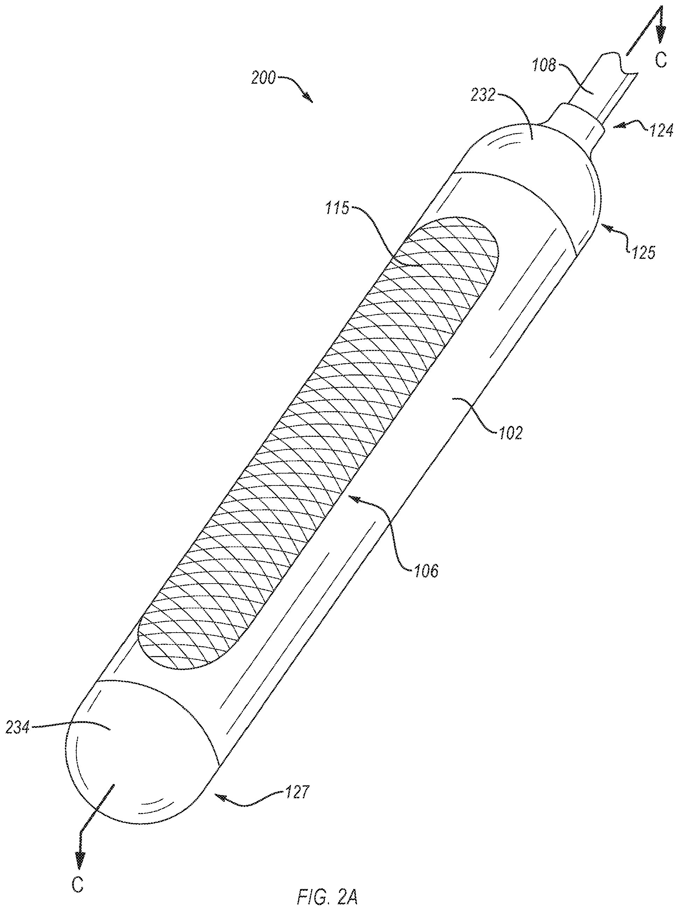

In some examples **(****FIGS. 2A-3B****),** the fluid permeable membrane 118 and the fluid permeable support may not substantially completely fill the portions of the chamber 104 that are not occupied by the conduit 108. In such examples, the fluid collection device 100 may include the reservoir (*e.g*., void space) disposed in the chamber 104. The reservoir may be a void space defined between the wicking material in the chamber 104 and the interior surface of the fluid impermeable coating 102 or an end cap **(****FIGS. 2A-3B****).** At least some of the fluid wicked by the wicking material 115 may drain out of the wicking material 115 and collect in the reservoir.

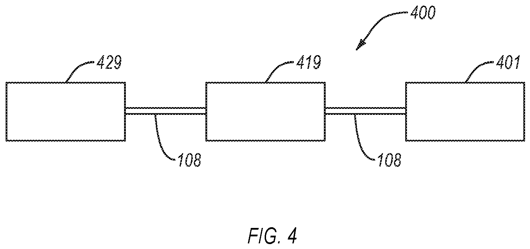

Returning to **FIGS. 1A** and **1B****,** the conduit 108 may be disposed within the wicking material 115 and includes the inlet 110 positioned within the fluid collection device 100 and the outlet for fluidly coupling (*e.g.*, plumbed or otherwise fluidly connected) to a vacuum source. The conduit 108 may include a flexible material such as plastic tubing *(e.g.,* medical tubing). Such plastic tubing may include a thermoplastic elastomer, polyvinyl chloride, ethylene vinyl acetate, polytetrafluoroethylene, etc., tubing. In examples, the conduit 108 may include silicon or latex. The conduit 108 includes an inlet 110 at the second end region 134 and an outlet 112 at a first end region 132 positioned downstream from the inlet 110. The inlet 110 of the conduit 108 fluidly couples the chamber 104 with a fluid storage container or a vacuum source for removing fluids from the fluid collection device 100. The fluid may be removed from the chamber 104 via the conduit 108. As suction or vacuum force is applied or formed in the conduit 108 by the vacuum source (**FIG. 4**), the fluid in the chamber 104 may be drawn into the inlet 110 and out of the fluid collection device 100 via the conduit 108.

In some examples, the inlet 110 may be disposed in an innermost or gravimetrically low spot in the chamber 104. For example, the conduit 108 may extend far enough into the chamber 104 to position the inlet 110 in a gravimetrically low point of the chamber 104 such as in a fluid reservoir within the chamber 104 the fluid collection device 100.

The fluid collection device 100 and components thereof may be deformable *(e.g.,* bendable) responsive to pressure applied thereto. For example, the fluid collection device 100 and the components thereof may bend to conform to the anatomical contours of the user, such as when disposed between a garment and the user. In examples, the fluid collection device 100 may bend when disposing proximate to the urethra (*e.g*., between the labia) when the fluid collection device 100 is positioned on a wearer (*e.g*., user).

The fluid collection device 100 is an example of a female fluid collection device 100 sized, shaped, and otherwise configured to receive fluids from a female user. For example, the fluid collection device 100 or at least one of the components therein may be substantially cylindrical, ellipsoid, prismatic, or any other shape suitable for complementing or contouring to the vaginal region of a female subject. The opening 106 may be configured to be positioned adjacent to a female urethra. The opening 106 may be positioned on an upward or inward (*e.g*., toward a user) facing portion of the fluid collection device 100.

In the illustrated example, the conduit 108 is at least partially disposed in the chamber 104. For example, the conduit 108 may extend into the fluid impermeable coating 102 from the first end region 125 and may extend to the second end region 127 to a point proximate to end of the fluid collection device 100 (portion of the device configured to be disposed at a gravimetrically low point of the device when in use). As shown in **FIG. 1B****,** the end of inlet 110 may be offset from the fluid impermeable coating 102 to allow fluid to be drawn therethrough. In some examples, the conduit 108 may be perforated, such as at the inlet end to allow radial collection of fluids from the wicking material. In some examples (not shown), the conduit 108 may enter the second end region 127 and the inlet 110 of the conduit 108 may be disposed in the second end region 127. The fluid collected in the fluid collection device 100 may be removed from the interior region of the chamber 104 via the conduit 108. The conduit 108 may include a flexible material such as plastic tubing (*e.g*., medical tubing) as disclosed herein. In some examples, the conduit 108 may include one or more portions that are resilient, such as to by having one or more of a diameter or wall thickness that allows the conduit to be flexible.

The fluid collection device 100 may be positioned proximate to the female urethra (e.g. on or between the labia) and urine may enter the chamber 104 of the fluid collection device 100 via the opening 106. The fluid collection device 100 receives the fluids into the chamber 104 via the opening 106. For example, the opening 106 may exhibit an elongated shape that is sized and positioned to extend from a first location below the urethral opening *(e.g.,* at or near the anus or the vaginal opening) to a second location above the urethral opening *(e.g.,* at or near the clitoris or the mons pubis). The opening 106 may exhibit an elongated shape since the space between the legs of a female is relatively small when the legs of the female of closed thereby only permitting the flow of the fluids along a path that corresponds to the elongated shape of the opening 106. The opening 106 in the fluid impermeable coating 102 may exhibit a width that is measured transverse to the longitudinal direction of the fluid collection device 100 and may be at least about 10% of the circumference of the fluid collection device 100, such as about 25%, 30%, 40%, 50%, 60%, 75%, 85%, 100% or ranges between any combination of the foregoing, of the circumference of the fluid collection device 100. The opening 106 may exhibit a width that is greater than 50% of the circumference of the fluid collection device 100 since the vacuum (*e.g.,* suction) pulls the fluid into the conduit 108.

In an example, one or more portions of the fluid impermeable coating 102 may be configured to be attached to a patient or a garment worn by a user, such as adhesively attached (*e.g.,* with a hydrogel adhesive, such as a hydrogel layer as disclosed in U.S. Patent No. 10,226,376) In some examples, the opening 106 may be longitudinally oriented (*e.g*., having a major axis parallel to the longitudinal axis of the device 100). In some examples, (not shown) the opening 106 may be laterally oriented (*e.g*., having a major axis perpendicular to the longitudinal axis of the device 100).

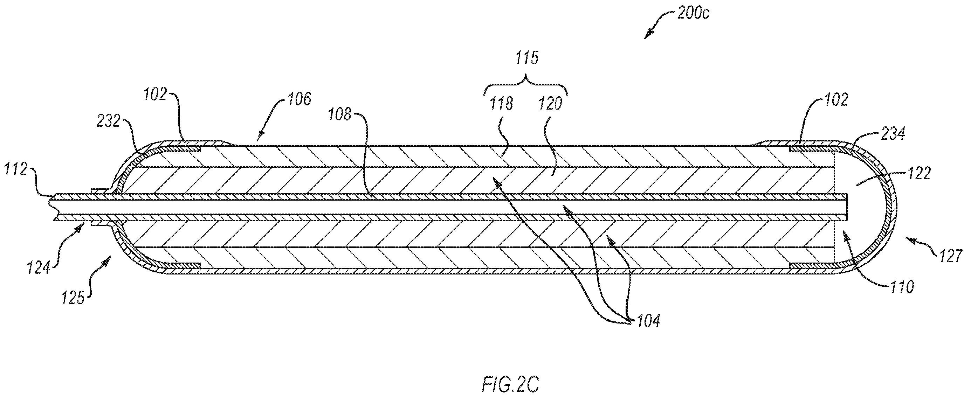

In an example, the conduit 108 is configured to be at least insertable into the chamber 104. In such an example, the conduit 108 may include one or more markers (not shown) on an exterior thereof that are configured to facilitate insertion of the conduit 108 into the chamber 104. For example, the conduit 108 may include one or more markings thereon that are configured to prevent over or under insertion of the conduit 108, such as when the conduit 108 defines an inlet 110 configured to be disposed in or adjacent to a reservoir in the fluid collection device (**FIGS. 2B** and **2C**). In another example, the conduit 108 may include one or more markings thereon that are configured to facilitate correct rotation or depth of insertion of the conduit 108 in the chamber 104. In an example, the one or more markings may include a line, a dot, a sticker, or any other suitable marking. In some examples, the conduit may be frosted or opaque (*e.g.,* black) to obscure visibility of the fluids therein.

Other examples of fluid impermeable materials, fluid permeable membranes, fluid permeable supports, chambers, and their shapes and configurations, which may be utilized in the examples described herein, are disclosed in U.S. Patent No. 16/369,676 filed on March 29, 2019 and U.S. Patent Application No. 15/260,103 filed on September 8, 2016 (published as US 2016-0374848 on December 29, 2016).

In some examples, the fluid collection device 100 may include a reservoir therein. In such examples, the fluid collection device may include one or more end caps configured to retain the first end region and the second end region of the wicking material therein.

**FIG. 2A** is an isometric view of a fluid collection device 200, according to an embodiment. **FIG. 2B** is a cross-sectional view of the fluid collection device of **FIG. 2A** taken along the plane C-C, according to an embodiment. **FIGS. 2A** and **2B** are described in detail below. The fluid collection device 200 may be similar or identical to the fluid collection device 100 in one or more aspects. For example, the fluid collection device 200 includes a fluid impermeable coating 102, wicking material 115, and conduit 108 as disclosed herein. The fluid collection device 200 includes at least one endcap, such as the first endcap 232 and the second endcap 234. The wicking material 115 may be disposed within the fluid impermeable coating 102. The at least one endcap may be disposed over the fluid impermeable coating 102 and the wicking material 115 at the first end region 125 and the second end region 127. Collectively, the fluid impermeable coating 102 and the at least one endcap define the chamber 104. The fluid collection device 200 may include a reservoir 122 within the chamber 104, such as a void space defined between the second endcap 234 and the wicking material 115. The fluid collection device 200 may also include the conduit 108. The conduit 108 may be at least partially disposed within wicking material 115 as disclosed herein.

As shown, the fluid impermeable coating 102 may be disposed over the wicking material 115 and the endcaps 232 and 234 may be disposed over the end regions of the coated wicking material. The endcaps may be sealed to the fluid impermeable coating, such as via a tension fit or a fluid impermeable adhesive. The first and second endcaps 232 and 234 may have a selected cross-sectional shape *(e.g.,* when viewed along the longitudinal axis), such as circular, elliptical, triangular, rectangular, or the like. The endcaps 232 and 234 may retain the wicking material 115 in the selected cross-sectional shape. For example, the first and second endcaps 232 and 234 may retain the wicking material 115 in a substantially cylindrical configuration as shown.

The first endcap 232 may be disposed over the wicking material 115 and fluid impermeable coating 102 at the first end region 125 of the fluid collection device 200. The first endcap 232 may include the aperture 124 sized and shaped to accept and seal against the conduit 108 passed therethrough.

The second endcap 234 may be disposed over the wicking material 115 and fluid impermeable coating 102 at the second end region 127. The second endcap 234 at least partially defines the reservoir 122 to collect fluid therein. The fluid impermeable coating 102 may also at least partially define the reservoir 122.

The at least one endcap may be formed of a fluid impermeable material. For example, the endcaps 232 and 234 may be made of the same or similar materials as the fluid impermeable coating 102. The at least one endcap *(e.g.,* second endcap 234) may include a more rigid material than the fluid impermeable coating 102, such as to allow the reservoir 122 to maintain a substantially constant volume when in use. The at least one endcap may be made of a resilient material to retain or return to a selected shape if deformed via external pressure.

The reservoir 122 may be located at the portion of the chamber 104 that is closest to the inlet 110 *(e.g.,* the second end region), such as under the second endcap 234. However, the reservoir 122 may be located at different locations in the chamber 104. For example, the reservoir 122 may be located at the end of the chamber 104 that is closest to the outlet 112, such as under the first endcap 232. In another example, fluid collection device 100 may include multiple reservoirs, such as a first reservoir that is located at the second end region 127 and a second reservoir that is located at the first end region 125. In another example, the fluid permeable support 120 is spaced from at least a portion of the conduit 108, and the reservoir 122 may be the space between the fluid permeable support 120 and the conduit 108.

As shown in **FIG. 2B****,** the inlet 110 of the conduit 108 may be coextensive with the end of the wicking material 115 at the second end region of the fluid collection device 200. In some examples, the inlet 110 of the conduit 108 may extend beyond the end of the wicking material 115 at the second end region of the fluid collection device 200. In some examples, the inlet 110 of the conduit 108 may be disposed within the wicking material 115 such as recessed inside of the channel in the wicking material 115 at the second end region of the fluid collection device 200.

In some examples, the one or more of the wicking material 115 and the fluid impermeable coating 102 at the first and/or second end regions of the fluid collection device 200 may have a stepped configuration selected to provide a substantially constant dimension (*e.g*., diameter) at the outer surface of the fluid collection device. Such a stepped configuration may include progressively smaller diameters at the second end region 127 of the wicking material 115 to accommodate the thickness of the fluid impermeable coating 102 and/or second endcap 234. Accordingly, the fluid collection device 200 may have a substantially smooth outer surface, free of cusps, gaps, or other discontinuities.

In some embodiments, the fluid impermeable coating 102 may be disposed over the wicking material 115 and the at least one endcap.

**FIG. 2C** is a side cross-sectional view of the fluid collection device of **FIG. 2A** taken along the plane C-C, according to an embodiment. **FIG. 2C** depicts the fluid collection device 200c. The fluid collection device 200c may be similar or identical to the fluid collection device 100 or 200 in one or more aspects. For example, the fluid collection device 200c includes one or more of the wicking material 115, the at least one endcap, wicking material 115, and the conduit 108 disposed within the fluid impermeable coating 102. As shown, the fluid impermeable coating 102 may be disposed over the wicking material 115 and the endcaps 232 and 234, collectively, such as via spray application, dip coating, direct application, etc. The endcaps 232 and 234 may be sealed in the fluid impermeable coating along with the wicking material 115. The endcaps and/or the fluid impermeable coating 102 thereover may aid in retaining the shape of the wicking material 115. In some examples, the fluid impermeable coating 102 may be disposed on a portion of the wicking material 115 *(e.g.,* to form the opening 106), the first endcap 232, and the second endcap 234. For example, the opening 106 may be formed by masking a portion of the wicking material 115 with a masking agent and dip coating the wicking material and first and second endcaps 232 and 234 in the fluid impermeable coating material. The masking agent may be removed from the wicking material to reveal the opening 106.

The first endcap 232 may be disposed over the wicking material 115 and within the fluid impermeable coating 102 at the first end region 125 of the fluid collection device 200c. The at least one endcap may be formed of a fluid impermeable material as disclosed above. The first endcap 232 may include the aperture 124 sized and shaped to accept and seal against the conduit 108 passed therethrough.

The second endcap 234 may be disposed over the wicking material 115 and within the fluid impermeable coating 102 at the second end region 127. The second endcap 234 at least partially defines the reservoir 122 to collect fluid therein. In such examples, the reservoir 122 may be a void space defined between the second endcap 234 and the wicking material 115.

As shown in **FIG. 2C****,** in some embodiments, the inlet 110 of the conduit 108 may extend past the end of the wicking material 115 at the second end region of the fluid collection device 200c. In some embodiments, the inlet 110 of the conduit 108 may be coextensive with the end of the wicking material 115 or may be recessed inside of the channel in the wicking material 115 at the second end region 127.

In some examples, wicking material 115 may have a stepped configuration selected to accommodate the thickness of one or more of the at least one endcap and the fluid impermeable coating 102 to provide a substantially smooth outer surface for the fluid collection device when the fluid impermeable coating is applied thereover. Such a stepped configuration may include progressively smaller diameters at one or more of the first and second end regions 125 and 127 of the wicking material 115 to accommodate the first and second endcap 232 and 234. Accordingly, the fluid collection device 200 may have a substantially smooth outer surface free of cusps, gaps, steps, or other discontinuities. By coating the wicking material 115 and the at least one endcap in the fluid impermeable coating 102, the fluid collection device 200c may be less prone to leakage than the fluid collection device 200 which includes transitions between materials at the outer surface.

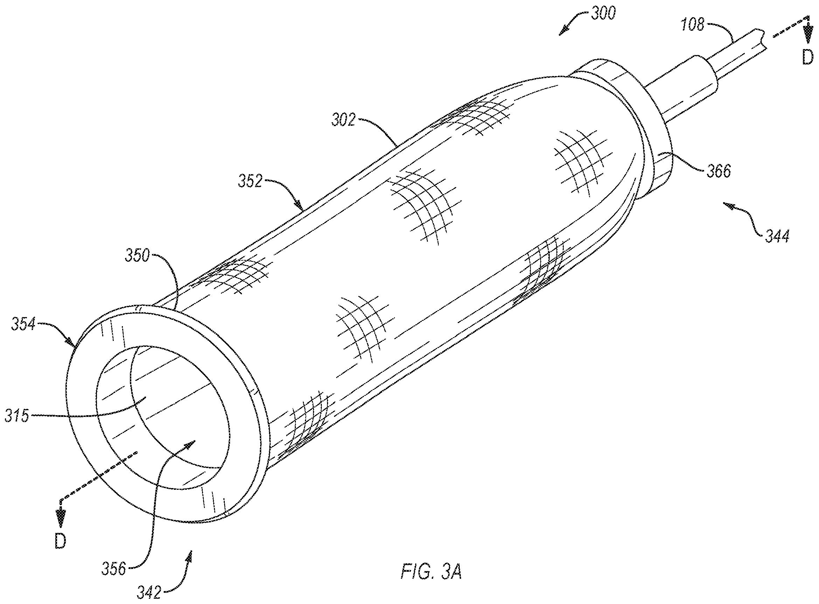

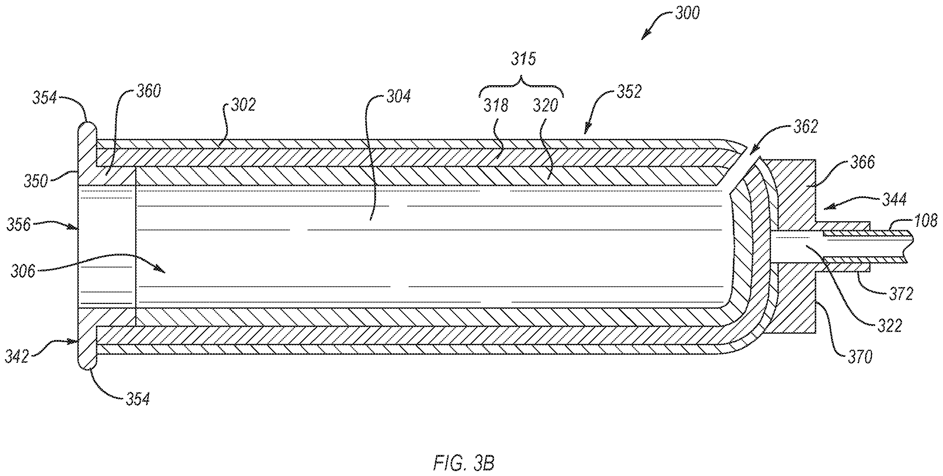

**FIG. 3A** is an isometric view of a fluid collection device 300, according to an embodiment. **FIG. 3B** is a cross-sectional view of a fluid collection device 300 of **FIG. 3A****,** according at least some embodiments. Referring to **FIGS. 3A** and **3B****,** the fluid collection device 300 includes a receptacle 350 and a sheath 352 *(e.g.,* cup shaped container). The receptacle 350 is sized, shaped, and made of a material to be disposed on the skin that surrounds the male urethra and have the male urethra positioned therethrough. For example, the receptacle 350 may include an annular base 354 that defines an opening 356 in the receptacle 350. The annular base 354 is sized and shaped to be positioned around the male urethra (*e.g*., positioned around the penis) and the opening 356 may be configured to have the male urethra positioned therethrough. The annular base 354 may also be sized, shaped, made of a material, or otherwise configured to be coupled (*e.g*., adhesively attached, such as with a hydrogel adhesive) to or disposed in a garment around the male urethra (*e.g*., around the penis). The receptacle 350 may be formed of any of the materials disclosed herein for the fluid impermeable coating 102, such as a silicone polymer. In an example, the annular base 354 may exhibit the general shape of the skin surface that the annular base 354 is selected to be coupled with and/or may be flexible thereby allowing the annular base 354 to conform to any shape of the skin surface. The receptacle 350 also defines a hollowed region that is configured to receive *(e.g.,* seal against) the sheath 352. For example, the receptacle 350 may include a flange 360 that extends longitudinally from the annular base 354. The flange 360 may be tall enough to prevent the sheath 352 from being accidentally removed from the receptacle 350 *(e.g.,* at least 0.5 cm tall, 1 cm tall, at least 2 cm tall, or at least 5 cm tall). While depicted as being disposed within the sheath 352, in some examples the flange 360 may be disposed on the outer surface of the sheath 352.

The sheath 352 includes *(e.g.,* may be formed from) a fluid impermeable coating 302 that is sized and shaped to fit into or around opening 356 *(e.g.,* the hollowed region) and/or flange 360 of the receptacle 350. The fluid impermeable coating 302 may be disposed on wicking material 315. The sheath 352 may be shaped to contain, and allow for, different sizes and states of male penises. For example, the sheath 352 may be generally cup-shaped or tubular with a substantially closed end. In some examples, the sheath 352 may be substantially cylindrical with a closed distal end. The cylindrical sheath 352 may be "substantially" cylindrical in that the cylinder may be deformable and at least partially collapsible/expandable based on the size and state of the penis of the wearer. Accordingly, the penis of a wearer may be inserted into and contained within the sheath 352 whether in a flaccid or erect state.

The fluid impermeable coating 302 may be similar or identical to the fluid impermeable coating 102, in one or more aspects. The fluid impermeable coating 302 at least partially defines a chamber 304 therein. The fluid impermeable coating 302 may also define an opening 306 extending through the fluid impermeable coating 302 that is configured to have a male urethra positioned therethrough. The fluid impermeable coating 302 may also include at least one aperture 362 that allows the chamber 304 to remain substantially at atmospheric pressure. The aperture 362 may be disposed on any portion of the sheath 352, such as a portion not intended to serve as a reservoir for collected fluids. In some examples (not shown), the aperture 362 may extend through the cap 366 or be disposed beneath the cap 366. In some examples, the fluid collection device 300 may not include the aperture 362, such as when a more complete seal as desired for the chamber 304.

The fluid impermeable coating 302 may be disposed on, or at least partially infiltrated in, an outer surface of the wicking material 315 to form a fluid impermeable barrier separating the interior (*e.g*., chamber 304) of the fluid collection device 300 from the external environment. The wicking material 315 may be similar or identical to the wicking material 115 disclosed herein, in one or more aspects. For example, the wicking material 315 includes one or more of the fluid permeable membrane 118 or the fluid permeable support 120 disclosed herein. In some examples, the fluid permeable membrane 118 or the fluid permeable support 120 may be omitted from the wicking material 315. The fluid impermeable coating 302 may be adhered to the outer surface of the wicking material 315 *(e.g.,* the fluid permeable membrane 118 or the fluid permeable support 120). Put another way, the fluid permeable support 120 may be disposed on an interior surface of the fluid impermeable coating 302. The fluid permeable membrane 118 may be disposed on an interior surface of the fluid permeable support 120, such as disposed concentrically within the fluid permeable support 120. In such examples, the fluid permeable membrane 118 may contact the skin of the wearer *(e.g.,* penis) when in use. In some examples (not shown), the fluid permeable membrane 118 may be disposed at the outer surface of the wicking material 315, wherein the fluid impermeable coating 302 is adhered to the outer surface of the fluid permeable membrane 118. One or more of the fluid permeable membrane 118 or the fluid permeable support 120 may extend around at least a portion of the inner surface of the fluid impermeable coating 302.

To facilitate fluid collection and provide comfort, the sheath 352 may be flexible, relatively soft, and have a relatively smooth outer surface, thereby allowing the sheath 352 to correspond to the shape of a penis. For example, the sheath 352 may at least partially collapse when the penis is flaccid and at least partially expand and bend to the shape of the penis as the penis becomes erect.

The receptacle 350 may be more rigid than the sheath 352. For example, the receptacle 350 may be formed from a flexible polymer that is at least one of thicker than the entire sheath 352 or exhibits a Young's modulus that is greater than sheath 352. As such, the receptacle 350 may provide some structural support at or near the proximal end 342 of the fluid collection device 300. The higher rigidity of the receptacle 350 may cause the receptacle 350 to remain open, thereby facilitating insertion of the urethra *(e.g.,* penis) into the fluid collection device 300. The relatively rigidity of the receptacle 350 enables the receptacle 350 to act as an attachment point to a wearer, wicking material 315, and/or the fluid impermeable coating 302. In some examples, the receptacle 350 may define a recess, include threads, or include any other attachment substrate for attaching the components of the fluid collection device 300, such as the wicking material 315 or the coating 302.

In some examples, the fluid collection device 300 includes a cap 366 at a distal end region 344. The cap 366 defines an interior channel through which the fluids may be removed from the fluid collection device 300. The interior channel is in fluid communication with the chamber 304. The cap 366 may be disposed over at least a portion of the distal end region 344 of one or more of the fluid impermeable coating 302 or the wicking material 315. The cap 366 may be made of a polymer, rubber, or other fluid impermeable material. The cap 366 may be attached to one or more of the fluid impermeable coating 302, the wicking material 315, or the conduit 108. The cap 366 may have a laterally extending flange 370 and a longitudinally extending flange 372. The laterally extending flange 370 may cover at least a portion of the distal end region 344 of the fluid collection device 300. The longitudinally extending flange 372 may extend a distance from the wicking material 315. The longitudinally extending flange 372 is sized and configured to receive and fluidly seal against the conduit 108, such as within the interior channel. The conduit 108 may extend a distance within the cap 366, such as to the wicking material 315, through the wicking material 315, or to a point set-off from the wicking material 315. In the latter example, as depicted in **FIG. 3B****,** the interior channel of the cap 366 may define a reservoir 322 therein. The reservoir 322 is an unoccupied portion of the cap 366 and is void of other material. In some examples, the reservoir 322 is defined at least partially by the wicking material 315 and the cap 366. During use, the fluids that are in the chamber 304 may flow through the wicking material 315 to the reservoir 322. The reservoir 322 may store at least some of the fluids therein and/or position the fluids for removal by the conduit 108. In some examples, at least a portion of the wicking material 315 may extend continuously between at least a portion of the opening of the interior channel and chamber 304 to wick any fluid from the opening directly to the reservoir 322.

In some examples (not shown), the fluid impermeable coating 302 may be disposed on the cap 366, such as enclosing the cap 366 within the chamber 304.

In some examples, the sheath 352 may include at least a portion of the conduit 108 therein, such as at least partially disposed in the chamber 304. For example, the conduit 108 may extend from the sheath 352 to a region at least proximate to the opening 356. The region proximate to the opening 356 may be disposed near or on the skin around the male urethra *(e.g.,* on the penis). Accordingly, when a patient lays on their back, fluid (*e.g.,* urine) may aggregate near the opening 306 against the skin of the wearer. The fluid may be removed from the chamber 304 via the conduit 108. The conduit 108 may be disposed within the wicking material and includes the inlet positioned within the fluid collection device and an outlet configured to be fluidly coupled to a vacuum source.

The receptacle 350, the sheath 352, the cap 366, and the conduit 108 may be attached together using any suitable method. For example, at least two of the receptacle 350, the sheath 352, the cap 366, or the conduit 108 may be attached together using at least one of an interference fit, an adhesive, stitching, welding (*e.g*., ultrasonic welding), tape, any other suitable method, or combinations thereof.

In some examples, the vacuum source (not shown) may be remotely located from the sheath 352. In such examples, the conduit 108 may extend out of and away from the sheath 352 to the vacuum source or a fluid storage container. For example, the inlet of the conduit may be used to remove fluid from the chamber 304 via vacuum when an outlet (not shown) of the conduit 108 is fluidly coupled to the vacuum source or fluid storage container operably coupled to the vacuum source.

In some examples (not shown), the fluid collection device 300 does not include the cap 366. In such examples, the fluid impermeable coating 302 may include at least one aperture sized and shaped to receive and seal against the conduit 108, such as within the chamber 304. In such examples, the outlet of the conduit 108 may be fluidly coupled to a fluid storage container or vacuum device (not shown). Accordingly, the interior region of

As the vacuum source (**FIG. 4**) applies a vacuum/suction in the conduit 108, the fluid(s) in the chamber 104 or 304 *(e.g.,* at the second end region 244 such as in the reservoir 322) may be drawn into the inlet and out of the fluid collection device 100, 200, or 300 via the conduit 108.

**FIG. 4** is a block diagram of a system 400 for fluid collection, according to an embodiment. The system 400 includes a fluid collection device 401, a fluid storage container 419, and a vacuum source 429. The fluid collection device 401, the fluid storage container 419, and the vacuum source 429 may be fluidly coupled to each other via one or more conduits 108. For example, fluid collection device 401 may be operably coupled to one or more of the fluid storage container 419 or the (portable) vacuum source 429 via the conduits 108. The fluid collection device 401 may be similar or identical to any of the fluid collection devices disclosed herein, such as a male or female fluid collection device.

Fluid *(e.g.,* urine or other bodily fluids) collected in the fluid collection device 401 may be removed from the fluid collection device 401 via the conduit 108 which protrudes into an interior region (*e.g*., chamber) of the fluid collection device 401. For example, a first open end of the conduit 108 may extend into the fluid collection device 401 to a reservoir therein. The second open end of the conduit 108 may extend into the fluid collection device 401 or the vacuum source 429. The suction force may be introduced into the interior region of the fluid collection device 401 via the first open end of the conduit 108 responsive to a suction (*e.g*., vacuum) force applied at the second end of the conduit 108. The suction force may be applied to the second open end of the conduit 108 by the vacuum source 429 either directly or indirectly.

The suction force may be applied indirectly via the fluid storage container 419. For example, the second open end of the conduit 108 may be fluid coupled to or disposed within the fluid storage container 419 and an additional conduit 108 may extend from the fluid storage container 419 to the vacuum source 429. Accordingly, the vacuum source 429 may apply suction to the fluid collection device 401 via the fluid storage container 419. As the fluid is drained from the chamber, the fluid may travel through the first section of conduit to the fluid storage container where it may be retained. Accordingly, fluid, such as urine, may be drained from the fluid collection device 401 using the vacuum source 429. In some examples, the suction force may be applied directly via the vacuum source 429. For example, the second open end of the conduit 108 may be disposed within the vacuum source 429. An additional conduit 108 may extend from the vacuum source 429 to a point outside of the fluid collection device 401, such as to the fluid storage container 419. In such examples, the vacuum source 429 may be disposed between the fluid collection device 401 and the fluid storage container 419.

In examples, the fluid storage container 419 may include a bag *(e.g.,* drainage bag), a bottle or cup *(e.g.,* collection jar), or any other enclosed container for storing bodily fluids such as urine.

The vacuum source 429 may include one or more of a manual vacuum pump, and electric vacuum pump, a diaphragm pump, a centrifugal pump, a displacement pump, a magnetically driven pump, a peristaltic pump, or any pump configured to produce a vacuum. The vacuum source 429 may provide a vacuum or suction to remove fluid from the fluid collection device 401. In examples, the vacuum source 429 may be powered by one or more of a power cord *(e.g.,* connected to a power socket), one or more batteries, or even manual power *(e.g.,* a hand operated vacuum pump). The vacuum sources 429 disclosed herein may include one or more of a switch, a button, a plug, a remote, or any other device suitable to activate the vacuum source 429.

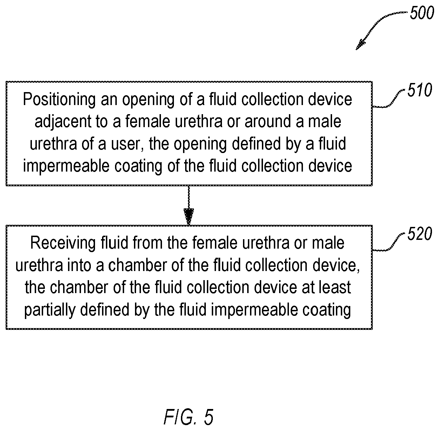

**FIG. 5** is a flow diagram of a method 500 to collect fluid, according to an embodiment. The method 500 may include act 510, which recites "positioning an opening of a fluid collection device adjacent to a female urethra or around a male urethra of a user, the opening defined by a fluid impermeable coating of the fluid collection device." Act 510 may be followed by act 520, which recites "receiving fluid from the female urethra or male urethra into a chamber of the fluid collection device, the chamber of the fluid collection device at least partially defined by the fluid impermeable coating."

Acts 510 and 520 of the method 500 are for illustrative purposes. For example, the act 510 and 520 of the method 500 may be performed in different orders, split into multiple acts, modified, supplemented, or combined. In an example, one or more of the acts 510 and 520 may be omitted from the method 500. For example, the method 500 may not include the act 510. Any of the acts 510 or 520 may include using any of the fluid collection devices, vacuum sources, fluid storage containers, systems, or components of the same disclosed herein. For example, the fluid collection device may include a fluid impermeable coating at least partially defining the chamber, the fluid impermeable coating also defining an opening extending therethrough, the opening configured to be positioned adjacent to a female urethra or have a male urethra positioned therethrough. The fluid collection device may include wicking material disposed with the chamber and a conduit disposed within the wicking material, the conduit including an inlet positioned within the fluid collection device and an outlet.

Act 510 recites "positioning an opening of a fluid collection device adjacent to a female urethra or around a male urethra of a user, the opening defined by a fluid impermeable coating of the fluid collection device." The fluid collection device may be a female fluid collection device, such as any of those disclosed herein. The fluid collection device may be a male fluid collection device, such as any of those disclosed herein. Positioning an opening of a fluid collection device adjacent to a female urethra of a user may include disposing the opening against, between, or inside the labia majora of the female user. Positioning an opening of a fluid collection device adjacent to a female urethra of a user may include disposing the second end region of the fluid collection device in the gluteal cleft of the female user. Positioning an opening of a fluid collection device adjacent to a female urethra or around a male urethra of a user may include securing the fluid collection device in position on the user, such as via tape, an adhesive, a wrap, inserting into clothing, etc. Positioning an opening of a fluid collection device around a male urethra of a user may include positioning the opening of the male fluid collection device around the penis of the user *(e.g.,* wearer). In such an example, the method 500 may include positioning a sheath of the male fluid collection device in a hollowed region of the receptacle such that the male urethra is positioned through an opening of the sheath of the male fluid collection device.

Act 520 recites "receiving fluid from the female urethra or male urethra into a chamber of the fluid collection device, the chamber of the fluid collection device at least partially defined by the fluid impermeable coating." For example, act 520 may include wicking the fluids away from the urethra, such as using wicking material *(e.g.,* fluid permeable membrane and a fluid permeable support) via the opening or chamber. The act 520 may include wicking the fluids away from the urethra such as to a reservoir in the fluid collection device. The act 520 may include flowing the fluid towards a portion of the chamber that is fluidly coupled to an inlet of a conduit for removing the fluid from chamber *(e.g.,* conduit in fluid communication a vacuum source). For instance, act 520 may include flowing the fluids to a substantially unoccupied portion of the chamber *(e.g.,* a reservoir), to a gravimetrically low point of the chamber, etc.

The method 500 may include applying suction with a vacuum source effective to suction the fluids from the chamber via a conduit disposed therein and fluidly connected to the vacuum source. In examples, applying suction with a vacuum source effective to suction the fluids from the chamber via a conduit disposed therein and fluidly coupled to the vacuum source may include using any of the vacuum sources disclosed herein. In an example, applying suction may include activating the vacuum source (*e.g*., suction device) in fluid communication with the inlet of the conduit in the fluid collection device. In examples, activating the vacuum source in fluid communication with the inlet of the conduit in the fluid collection device may include supplying power to the vacuum source by one or more of flipping an on/off switch, pressing a button, plugging the vacuum source into a power outlet, putting batteries into the vacuum source, etc. In examples, the vacuum source may include a hand operated vacuum pump and applying suction with a vacuum source may include manually operating the hand operated vacuum pump effective to suction the fluids from the chamber.

In examples, applying suction with a vacuum source effective to suction the fluids from the chamber may be effective to remove at least some fluid *(e.g.,* urine) from the chamber of the fluid collection device. In examples, applying suction with a vacuum source effective to suction the fluids from the chamber via a conduit disposed therein and fluidly coupled to the vacuum source may be effective to transfer at least some of the fluid from the chamber of the fluid collection device to a fluid storage container *(e.g.,* a bottle or bag) fluidly coupled thereto. In examples, the vacuum source may be spaced from the fluid collection device.

In examples, applying suction with a vacuum source effective to suction the fluids from the chamber via a conduit disposed therein and fluidly coupled to the vacuum source may include detecting moisture in the chamber (*e.g*., via one or more moisture sensors) and responsive thereto, activating the vacuum source to provide suction in the chamber. The control of the vacuum source responsive to the signals indicating that moisture or a level thereof is present in the chamber may be automatic, such as via a controller, or may merely provide an indication that a level of moisture is present that may necessitate removal of fluid from the chamber of the fluid collection device. In the latter case, a user may receive the indication and activate the vacuum pump.

In an example, the method 500 may include collecting the fluids that are removed from the fluid collection device, such as into a fluid storage container that is spaced from the fluid collection device and fluidly coupled to the conduit. The fluid storage container may include any of the fluid storage containers disclosed herein.

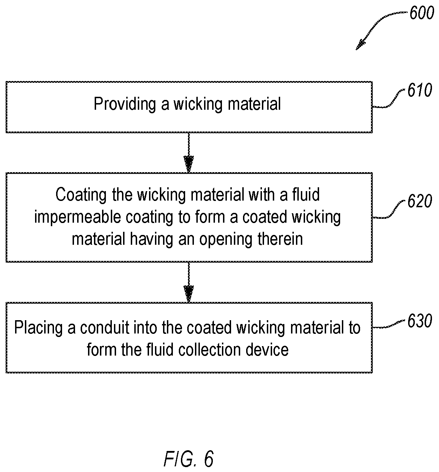

**FIG. 6** is a flow diagram of a method 600 to making a fluid collection device, according to an embodiment. The method 600 may include act 610, which recites "providing a wicking material." Act 610 may be followed by act 620, which recites "coating the wicking material with a fluid impermeable coating to form a coated wicking material having an opening therein." Act 620 may be followed by act 630, which recites "placing a conduit into the coated wicking material to form the fluid collection device."

Acts 610, 620, and 630 of the method 600 are for illustrative purposes. For example, the act 610, 620, and 630 of the method 600 may be performed in different orders, split into multiple acts, modified, supplemented, or combined. In an example, one or more of the acts 610, 620, and 630 of the method 600 may be omitted from the method 600. For example, the method 600 may not include the act 630. Any of the acts 610, 620, or 630 may include using any of the fluid collection devices, vacuum sources, fluid storage containers, systems, or components of the same disclosed herein. For example, the fluid impermeable coating at least partially defines the chamber, an opening extending therethrough, where the opening is sized and shaped to be positioned adjacent to a female urethra or have a male urethra positioned therethrough. The fluid collection device formed by the method 600 may include any of the fluid collection devices disclosed herein. For example, the fluid collection device may include wicking material disposed with the chamber and a conduit disposed within the wicking material, the conduit including an inlet positioned within the fluid collection device and an outlet.

Act 610 recites "providing a wicking material." Providing a wicking material may include providing any of the wicking materials in any of the configurations disclosed herein. For example, providing the wicking material may include providing a cylindrically shaped wicking material, such one or more of a cylindrically shaped fluid permeable membrane or a fluid permeable support. Providing the wicking material may include providing the wicking material of any of the female fluid collection devices disclosed herein. For example, the fluid permeable support may be concentrically disposed in the fluid permeable membrane. The fluid permeable support may include a channel formed therethrough to accommodate the conduit.

Providing the wicking material may include providing the wicking material of any of the male fluid collection devices disclosed herein. For example, providing the wicking material may include providing the fluid permeable membrane concentrically disposed within the fluid permeable support. The fluid permeable support and fluid permeable membrane may have a channel formed therein to allow a conduit to be inserted therein.

Act 620 recites "coating the wicking material with a fluid impermeable coating to form a coated wicking material having an opening therein." Coating the wicking material with a fluid impermeable coating to form a coated wicking material having an opening therein includes applying the coating onto the wicking material effective to coat or impregnate the outer surface of the wicking material with the fluid impermeable coating material to form the opening in the fluid impermeable coating. The opening may be as described herein for the female fluid collection device or the male fluid collection device.

Coating the wicking material with a fluid impermeable coating to form a coated wicking material having an opening therein may include utilizing any of the fluid impermeable coating materials disclosed herein in any of the thicknesses, surface characteristics, or shapes disclosed herein. Coating the wicking material with a fluid impermeable coating to form a coated wicking material having an opening therein may include one or more of dipping *(e.g.,* dip coating), spraying *(e.g.,* spray coating), wrapping *(e.g.,* shrink wrapping), brush application, rolling, casting, impregnation *(e.g.,* vacuum infiltration), or the like. For example, the opening may be formed by masking a portion of the wicking material with a masking agent and dip coating the wicking material (and optionally, the first and second endcaps) in the fluid impermeable coating material. After the fluid impermeable coating material dries, the masking agent may be removed from the wicking material to reveal the opening. In some examples, coating the wicking material with a fluid impermeable coating to form a coated wicking material having an opening therein may include coating only the portions of the wicking material (or endcaps) with the fluid impermeable coating material outside of a selected dimension and location of selected opening. Accordingly, selective application of the fluid impermeable coating material may be used to form the opening.

Act 630 recites "placing a conduit into the coated wicking material to form the fluid collection device." Placing a conduit into the coated wicking material to form the fluid collection device may include inserting the conduit into the coated wicking material to a distance therein. For example, the conduit may be inserted into and through the wicking material to a reservoir in the chamber of the fluid collection device. In some examples, placing a conduit into the coated wicking material may include disposing the conduit into the wicking material to a point within the wicking material or until the inlet of the conduit is co-extensive with the wicking material. Placing a conduit into the coated wicking material may include inserting the conduit through the coating.

Placing a conduit into the coated wicking material to form the fluid collection device may include disposing a conduit within the coated wicking material sufficient to form any of the fluid collection devices disclosed herein, such as a female fluid collection device or male fluid collection device.

In some examples, prior to coating, the method 600 may include disposing an endcap on one or more ends of the wicking material, such as placing any of the endcaps or caps on any of the ends of the coated wicking materials disclosed herein. For example, the method 600 may include placing an endcap on each end of a fluid permeable membrane disposed on a cylindrical fluid permeable support. Placing the endcap on the second end of a coated wicking material may form a reservoir in the chamber, such as between the endcap and the wicking materials. In such examples, the coating may be disposed over or under the endcaps or caps. In some examples, disposing an endcap on one or more ends of the wicking material may be carried out after coating at least a portion of the wicking material with the coating. In some examples, disposing the endcap on one or more ends of the wicking material may include adhering the endcaps to one or more of the wicking material or the fluid impermeable coating.

In some examples, the method 600 may include connecting, indirectly or directly, the conduit of the fluid collection device to a vacuum source. For example, connecting the conduit of the fluid collection device to a vacuum source may include connecting the conduit to the fluid storage container. Connecting the conduit of the fluid collection device to a vacuum source may include forming any of the systems disclosed herein.

As used herein, the term "about" or "substantially" refers to an allowable variance of the term modified by "about" by ±10% or ±5%. Further, the terms "less than," "or less," "greater than", "more than," or "or more" include as an endpoint, the value that is modified by the terms "less than," "or less," "greater than," "more than," or "or more."

While various aspects and embodiments have been disclosed herein, other aspects and embodiments are contemplated. The various aspects and embodiments disclosed herein are for purposes of illustration and are not intended to be limiting.

## Claims

1. A fluid collection device (200), comprising:
at least one wicking material (115) including an outer surface;
a fluid impermeable coating (102) adhered to at least a portion of the outer surface of the at least one wicking material, the fluid impermeable coating defining a chamber within the fluid collection device partially enclosing the at least one wicking material and defining an opening (106) through which a portion of the at least one wicking material is exposed, the opening configured to be positioned adjacent to a female urethra;
wherein the fluid collection device is substantially cylindrical;
wherein the at least one wicking material is disposed in a substantially cylindrical configuration;
the fluid collection device further comprising a first endcap (232) disposed over a first end of the cylindrical configuration and a second endcap (234) disposed over a second end of the cylindrical configuration, wherein the first endcap and the second endcap retain the wicking material in the cylindrical configuration; and further wherein:
the endcaps (232, 234) are sealed in the fluid impermeable coating along with the wicking material.

2. The fluid collection device of claim 1, wherein the at least one wicking material includes one or more of a fluid permeable membrane or a fluid permeable support.

3. The fluid collection device of any of claims 1-2, wherein the fluid impermeable coating is adhered to an outermost portion of the fluid permeable membrane.

4. The fluid collection device of any of claims 1-2, wherein the fluid impermeable coating is embedded within an outermost portion of the fluid permeable membrane.

5. The fluid collection device of any of claims 1-4, wherein the fluid impermeable coating includes one or more of a polymer, a wax, nanocellulose, or nanoparticles.

6. The fluid collection device of any of claims 1-5, wherein the fluid impermeable coating includes a superhydrophobic material.

7. The fluid collection device as claimed in any one of the preceding claims, wherein the second endcap (234) defines a reservoir (122) and includes a more rigid material than the fluid impermeable coating (102) such as to allow the reservoir to maintain a substantially constant volume when in use.

8. The fluid collection device of any of claims 1-7, further comprising a conduit (108) disposed within the chamber, the conduit including an inlet (110) positioned within the chamber and an outlet (112) configured to be fluidly coupled to a vacuum source (429).

9. A fluid collection system, comprising:
a fluid storage container (419) configured to hold a fluid;
a fluid collection device as claimed in claim 8; and
a vacuum source (429) fluidly coupled to one or more of the fluid storage container or the outlet of the fluid collection device, the vacuum source configured to draw fluid from the fluid collection device.

## Patentansprüche

1. Flüssigkeitssammelvorrichtung (200), umfassend:
mindestens ein Dichtmaterial (115), das eine Außenoberfläche einschließt;
eine für Flüssigkeiten undurchlässige Beschichtung (102), die mindestens an einen Abschnitt der Außenoberfläche des mindestens einen Dichtmaterials angehaftet ist, wobei die für Flüssigkeiten undurchlässige Beschichtung eine Kammer innerhalb der Flüssigkeitssammelvorrichtung definiert, die teilweise das mindestens eine Dichtmaterial umgibt und eine Öffnung (106) definiert, durch die ein Abschnitt des mindestens einen Dichtmaterials freigelegt ist, wobei die Öffnung ausgelegt ist, um angrenzend zu einer weiblichen Urethra positioniert zu sein;
wobei die Flüssigkeitssammelvorrichtung im Wesentlichen zylinderförmig ist;
wobei das mindestens eine Dichtmaterial in einer im Wesentlichen zylinderförmigen Konfiguration angeordnet ist;
wobei die Flüssigkeitssammelvorrichtung weiter eine erste Abschlusskappe (232) umfasst, die über einem ersten Ende der zylinderförmigen Konfiguration angeordnet ist, und eine zweite Abschlusskappe (234), die über einem zweiten Ende der zylinderförmigen Konfiguration angeordnet ist, wobei die erste Abschlusskappe und die zweite Abschlusskappe das Dichtmaterial in der zylinderförmigen Konfiguration zurückhalten; und wobei weiter:
die Abschlusskappen (232, 234) in der für Flüssigkeiten undurchlässigen Beschichtung zusammen mit dem Dichtmaterial versiegelt sind.

2. Flüssigkeitssammelvorrichtung nach Anspruch 1, wobei das mindestens eine Dichtmaterial eines oder mehreres einschließt von einer für Flüssigkeiten durchlässigen Membran oder einem für Flüssigkeiten durchlässigen Träger.

3. Flüssigkeitssammelvorrichtung nach einem der Ansprüche 1-2, wobei die für Flüssigkeiten undurchlässige Beschichtung an einem äußersten Abschnitt der für Flüssigkeiten durchlässigen Membran angehaftet ist.

4. Flüssigkeitssammelvorrichtung nach einem der Ansprüche 1-2, wobei die für Flüssigkeiten undurchlässige Beschichtung innerhalb eines äußersten Abschnitts der für Flüssigkeiten durchlässigen Membran eingebettet ist.

5. Flüssigkeitssammelvorrichtung nach einem der Ansprüche 1-4, wobei die für Flüssigkeiten undurchlässige Beschichtung eines oder mehreres einschließt von einem Polymer, einem Wachs, Nanocellulose oder Nanopartikeln.

6. Flüssigkeitssammelvorrichtung nach einem der Ansprüche 1-5, wobei die für Flüssigkeiten undurchlässige Beschichtung ein superhydrophobes Material einschließt.

7. Flüssigkeitssammelvorrichtung nach einem der vorstehenden Ansprüche, wobei die zweite Abschlusskappe (234) ein Reservoir (122) definiert und ein starreres Material als die für Flüssigkeiten undurchlässige Beschichtung (102) einschließt, um es zuzulassen, dass das Reservoir in Benutzung ein im Wesentlichen konstantes Volumen aufrechterhält.

8. Flüssigkeitssammelvorrichtung nach einem der Ansprüche 1-7, weiter umfassend eine Leitung (108), die innerhalb der Kammer angeordnet ist, wobei die Leitung einen Einlass (110), der innerhalb der Kammer positioniert ist, und einen Auslass (112), der ausgelegt ist, um flüssigkeitsmäßig mit einer Vakuumquelle (429) gekoppelt zu sein, einschließt.

9. Flüssigkeitssammelsystem, umfassend:
einen Flüssigkeitsspeicherbehälter (419), der ausgelegt ist, um eine Flüssigkeit zu halten;
eine Flüssigkeitssammelvorrichtung nach Anspruch 8; und
eine Vakuumquelle (429), die flüssigkeitsmäßig mit einem oder mehreren des Flüssigkeitsspeicherbehälters oder des Auslasses der Flüssigkeitssammelvorrichtung gekoppelt ist, wobei die Vakuumquelle ausgelegt ist, um Flüssigkeit aus der Flüssigkeitssammelvorrichtung zu ziehen.

## Revendications

1. Dispositif de collecte de fluide (200), comprenant :
au moins un matériau absorbant (115) incluant une surface extérieure ;
un revêtement imperméable aux fluides (102) collé à au moins une partie de la surface extérieure de l'au moins un matériau absorbant, le revêtement imperméable aux fluides définissant une chambre au sein du dispositif de collecte de fluide enfermant partiellement l'au moins un matériau absorbant et définissant une ouverture (106) à travers laquelle une partie de l'au moins un matériau absorbant est exposée, l'ouverture étant configurée pour être positionnée de manière adjacente à un urètre féminin ;
dans lequel le dispositif de collecte de fluide est sensiblement cylindrique ;
dans lequel l'au moins un matériau absorbant est disposé dans une configuration sensiblement cylindrique ;
le dispositif de collecte de fluide comprenant en outre un premier bouchon (232) disposé au-dessus d'une première extrémité de la configuration cylindrique et un second bouchon (234) disposé au-dessus d'une seconde extrémité de la configuration cylindrique, dans lequel le premier bouchon et le second bouchon maintiennent le matériau absorbant dans la configuration cylindrique ; et en outre dans lequel :
les bouchons (232, 234) sont scellés dans le revêtement imperméable aux fluides conjointement avec le matériau absorbant.

2. Dispositif de collecte de fluide selon la revendication 1, dans lequel l'au moins un matériau absorbant inclut un ou plusieurs parmi une membrane perméable aux fluides ou un support perméable aux fluides.

3. Dispositif de collecte de fluide selon l'une quelconque des revendications 1-2, dans lequel le revêtement imperméable aux fluides est collé à une partie de la membrane perméable aux fluides la plus à l'extérieur.

4. Dispositif de collecte de fluide selon l'une quelconque des revendications 1-2, dans lequel le revêtement imperméable aux fluides est incorporé au sein d'une partie de la membrane perméable aux fluides la plus à l'extérieur.

5. Dispositif de collecte de fluide selon l'une quelconque des revendications 1-4, dans lequel le revêtement imperméable aux fluides inclut un ou plusieurs parmi un polymère, une cire, la nanocellulose ou des nanoparticules.

6. Dispositif de collecte de fluide selon l'une quelconque des revendications 1-5, dans lequel le revêtement imperméable aux fluides inclut un matériau superhydrophobe.

7. Dispositif de collecte de fluide selon l'une quelconque des revendications précédentes, dans lequel le second bouchon (234) définit un réservoir (122) et inclut un matériau plus rigide que le revêtement imperméable aux fluides (102) de manière à permettre au réservoir de maintenir un volume sensiblement constant lors de l'utilisation.

8. Dispositif de collecte de fluide selon l'une quelconque des revendications 1-7, comprenant en outre un conduit (108) disposé au sein de la chambre, le conduit incluant une entrée (110) positionnée au sein de la chambre et une sortie (112) configurée pour être couplée de manière fluide à une source sous vide (429).

9. Système de collecte de fluide, comprenant :
un récipient de stockage de fluide (419) configuré pour retenir un fluide ;
un dispositif de collecte de fluide selon la revendication 8 ; et
une source sous vide (429) couplée de manière fluidique à un ou plusieurs parmi le récipient de collecte de fluide ou la sortie du dispositif de collecte de fluide, la source sous vide étant configurée pour aspirer un fluide depuis le dispositif de collecte de fluide.
